# EUROPEAN PATENT APPLICATION

(11) **EP 4 740 914 A1**
(43) Date of publication of application: **13.05.2026**
(21) Application number: 24835288.2
(22) Date of filing: 28.06.2024
(51) Int. Cl.: A61F 2/90

(54) **VASCULAR STENT**

(30) Priority: 06.07.2023 CN 202310827843
(71) Applicant: Lifetech Scientific (Shenzhen) Co., Ltd., Shenzhen, Guangdong 518063 (CN)
(72) Inventor: SHU, Chang, Shenzhen, Guangdong 518063 (CN); DAI, Shuang, Shenzhen, Guangdong 518063 (CN); XIAO, Benhao, Shenzhen, Guangdong 518063 (CN); RUI, Shixuan, Shenzhen, Guangdong 518063 (CN)
(74) Representative: Michalski Hüttermann & Partner Patentanwälte mbB
(86) International application number: PCT/CN2024/102353
(87) International publication number: WO 2025/007807

(57) **Abstract**

Disclosed in the present invention is a vascular stent. The vascular stent includes a main stent provided with a main lumen; the main stent includes a proximal section, a distal section, and a middle section located between the proximal section and the distal section and extending axially; a recessed portion is provided on the middle section; a supporting structure which protrudes outwards relative to the recessed portion is provided above the recessed portion; and at least part of the supporting structure is degradable. According to the vascular stent of the present invention, at least part of the supporting structure at the top of the vascular stent is configured to be degradable. In this way, the initial mechanical supporting effect of a recess section is guaranteed while retaining the overall structure. In a long term, the material of this part can be automatically absorbed after a certain time, thereby reducing the restraint and compression on a branch stent, reducing the occupation of the main lumen space, and reducing irritation to vascular wall.

## Description

### Technical Field

The present invention relates to the technical field of interventional medical devices, and particularly, to a vascular stent.

### Background Art

Aortic aneurysms and aortic dissections are severe life-threatening conditions. Without active treatment, aortic aneurysm sacs and aortic dissections will progressively enlarge and may eventually rupture, leading to severe complications and mortality. With the increasing prevalence of hypertension, hyperlipidemia, and hyperglycemia, the incidence of aortic aneurysms and aortic dissections has also risen significantly.

Traditional open surgery for treating aortic aneurysms and aortic dissections is associated with a large surgical incision, elevated mortality rates, prolonged operation times, high postoperative complication rates, and considerable surgical difficulty. In contrast, endovascular treatment has become a preferred method due to its features of a minimal incision, fewer postoperative complications, shorter operation time, and reduced surgical complexity. The endovascular treatment involves implanting a covered stent within the aorta to isolate a diseased vascular segment outside the covered stent and direct blood flow through a lumen of the covered stent, thereby protecting the vessel.

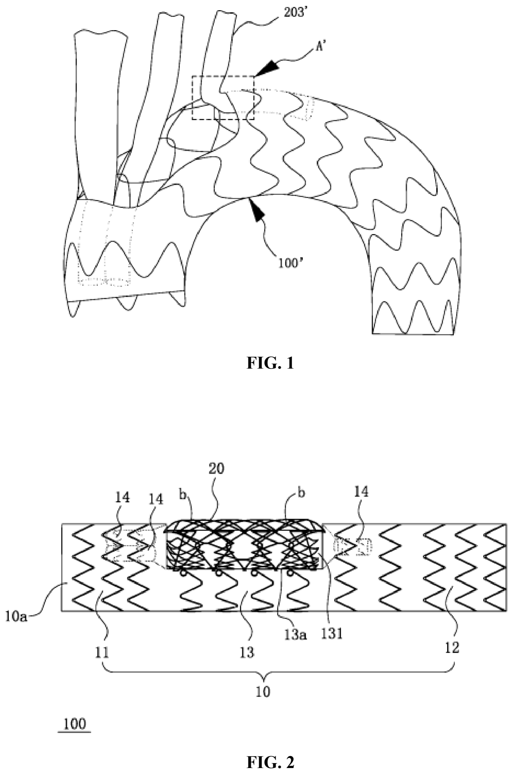

As shown in FIG. 1, a supporting corrugated ring of an existing aortic covered stent 100' is generally made of a memory metal alloy to facilitate the selection of the brachiocephalic artery, the left common carotid artery, and the left subclavian artery through an internal branch by using a guidewire during surgery. However, after being implanted, a branch stent will occupy corresponding main lumen space due to the restraint of the supporting corrugated ring, and the supporting corrugated ring exerts a negative compressive effect on the implanted branch stent. In particular, as indicated at position A' in FIG. 1, a branch stent 203' corresponding to the left subclavian artery is prone to restraint and compression by the supporting corrugated ring after being implanted, which may hinder blood flow through the left subclavian artery. Furthermore, the presence of the supporting corrugated rings may cause long-term irritation to the vascular wall or induce a retrograde tear in the vascular wall.

### Summary of the Invention

In view of the above, the present invention provides a vascular stent to solve at least part of the aforementioned technical problems.

To achieve this objective, the present invention adopts the following technical solutions.

The present invention provides a vascular stent, including a main stent provided with a main lumen. The main stent includes a proximal section, a distal section, and a middle section located between the proximal section and distal section and extending axially. The middle section is provided with a recessed portion, above which a supporting structure is arranged. At least part of the supporting structure is degradable.

In one embodiment, the supporting structure is provided with a branch passage that radially penetrates through the supporting structure. The branch passage allows a branch stent to pass through. At least part of an edge of the branch passage is degradable, enabling the branch passage to be expanded upon at least partial degradation. The expanded branch passage exerts reduced compression on the branch stent passing therethrough.

In one embodiment, the branch passage includes at least a right branch passage near a distal side of the supporting structure; and/or a left branch passage near a proximal side of the supporting structure.

In one embodiment, the supporting structure includes a mesh structure having a plurality of grids, and one or more of the grids form the branch passage.

In one embodiment, the supporting structure includes a main supporting layer that is at least partially degradable. The main supporting layer is formed by supporting filaments, and the supporting filaments include at least a degradable first supporting filament.

In one embodiment, the degradable first supporting filament is either entirely degradable or partially degradable. When the degradable first supporting filament is partially degradable, the first supporting filament has a multi-layer structure arranged from inside to outside, and includes a non-degradable inner core located at an innermost layer and a degradable covering layer located at an outermost layer.

In one embodiment, the degradable first supporting filament forms the main supporting layer.

In one embodiment, the supporting filaments further include a non-degradable second supporting filament, and the degradable first supporting filament and the non-degradable second supporting filament jointly form the main supporting layer.

In one embodiment, the first supporting filament forms a degradable layer, while the second supporting filament forms a non-degradable layer, and the degradable layer and the non-degradable layer are arranged opposite to each other within a same layer; or
the first supporting filament forms a degradable layer, while the second supporting filament forms a non-degradable layer, and the degradable layer and the non-degradable layer are adjacent to and connected to each other at an adjacent position; or
the first supporting filament and the second supporting filament are arranged in an interlaced manner within a same layer, such that the first supporting filament forms a plurality of degradable layers, while the second supporting filament forms a non-degradable layer, and the plurality of degradable layers are distributed in the non-degradable layer within the same layer.

In one embodiment, during the formation of the main supporting layer, the plurality of supporting filaments are intersected and form intersection points; and at the intersection points, the plurality of supporting filaments are interconnected, and/or overlapped, and/or hooked.

In one embodiment, when the plurality of supporting filaments are overlapped at the intersection points, the plurality of supporting filaments at the intersection points may be connected via a fastener and the fastener is configured to radially restrain the plurality of supporting filaments to avoid radial separation from one another; and/or
when the plurality of supporting filaments include the first supporting filament and the second supporting filament, and the first supporting filament and the second supporting filament are overlapped at the intersection points, the first supporting filament at the intersection points is located above the second supporting filament; and/or
when the plurality of supporting filaments include the first supporting filament and the second supporting filament, and the first supporting filament and the second supporting filament are overlapped at the intersection point, a radial thickness of the first supporting filament at least at the intersection point is greater than a radial thickness of the second supporting filament, and a cross passage through which the second supporting filament passes is formed at the intersection point of the first supporting filament.

In one embodiment, when the supporting filaments at the intersection point are connected with each other via the fastener, a first sliding passage extending in a first predetermined direction and a second sliding passage extending in a second predetermined direction are formed on the fastener. One of the supporting filaments slides relative to the fastener along the first predetermined direction, while the other supporting filament slides relative to the fastener along the second predetermined direction. The first predetermined direction and the second predetermined direction are predetermined movement directions of the supporting filaments when the vascular stent is compressed or self-expands; and/or
when the cross passage through which the second supporting filament passes is formed on the first supporting filament, the cross passage has a predetermined length along an extension direction of the first supporting filament.

In one embodiment, when the plurality of supporting filaments are hooked, an axially extending elastic connecting member is arranged between the supporting filaments at a hooking position. End portions of the elastic connecting member are respectively connected to hooked portions of the plurality of supporting filaments. The elastic connecting member is configured to elastically pull the supporting filaments toward each other.

In one embodiment, the first supporting filament may be formed by three-dimensional (3D) printing, and/or injection molding, and/or metal cutting, and/or braiding; and/or, when the supporting filaments include the second supporting filament, the second supporting filament may be formed by metal cutting and/or braiding.

In one embodiment, the supporting structure further includes a non-degradable local supporting layer; and the local supporting layer and the main supporting layer are stacked from inside to outside. The local supporting layer is opposite to the degradable layer that is formed by the first supporting filament and that is located in the main supporting layer.

In one embodiment, the main supporting layer includes a mesh structure. In naturally expanded state, the main supporting layer includes, in a circumferential direction, a first mesh region and a second mesh region connected to the first mesh region. The first mesh region includes a plurality of rows of intersection units which are formed by mutually overlapping a plurality of first-direction supporting filaments arranged at intervals and a plurality of second-direction supporting filaments arranged at intervals. The second mesh region includes at least one row of hooking units, each row of hooking units includes one or more axially arranged hooking units. Each hooking unit includes a first hooking member and a second hooking member. In at least part of the hooking units, the first hooking members and the second hooking members are substantially hooked with each other in an axial direction. Alternatively, in at least part of the hooking units, the first hooking members and the second hooking members are substantially separated in an axial direction to form a gap. Alternatively, in at least part of the hooking units, the first hooking members and the second hooking members are abutted against each other in a non-hooking manner.

In one embodiment, a degradable material includes a degradable polymer material and/or a degradable metal material.

In one embodiment, the degradable polymer material includes at least one of polylactic acid, polyglycolic acid, polybutylene succinate, poly(β-hydroxybutyrate), poly(β-alkanoate), polycaprolactone, poly(ethylene adipate), polylactic-co-glycolic acid, polyhydroxybutyrate-co-hydroxyvalerate, polyvinyl alcohol, polyethylene glycol, polyvinylpyrrolidone, starch, cellulose, polyethylene oxide, pectin, collagen, chitosan, dextran, chitin, and derivatives of the above polymers; or a copolymer formed by copolymerization of at least two monomers that constitute the aforementioned materials; and/or
the degradable metal material includes at least one of zinc, magnesium, iron, a zinc-based alloy, a magnesium-based alloy, and an iron-based alloy.

According to the vascular stent of the present invention, at least part of the supporting structure at the top of the vascular stent is configured to be degradable. In this way, the initial mechanical supporting effect of a recess section is guaranteed while retaining the overall structure. In a long term, the material of this part can be automatically absorbed after a certain time, thereby reducing the restraint and compression on a branch stent, reducing the occupation of the main lumen space, and reducing irritation to vascular wall.

### Brief Description of the Drawings

FIG. 1 is a schematic structural diagram of an aortic covered stent for implantation in the prior art.
FIG. 2 is a schematic structural diagram of an exemplary vascular stent according to the present invention.

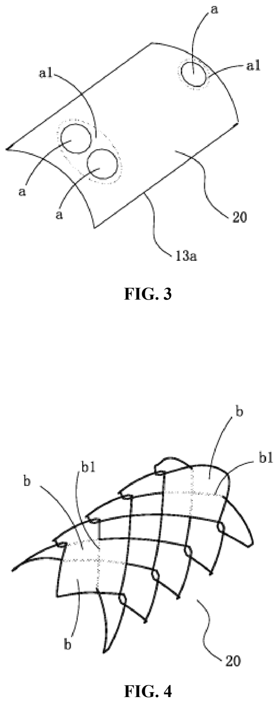

FIG. 3 is a schematic structural diagram of a supporting structure of an exemplary vascular stent according to the present invention.
FIG. 4 is schematic structural diagram of another supporting structure of an exemplary vascular stent according to the present invention.

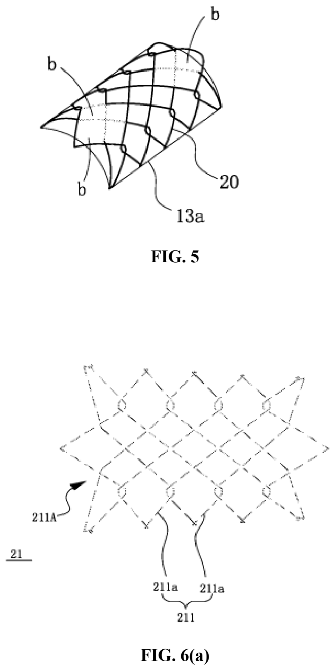

FIG. 5 is a schematic diagram of a connection between a supporting structure and an edge of a recessed portion of an exemplary vascular stent according to the present invention.

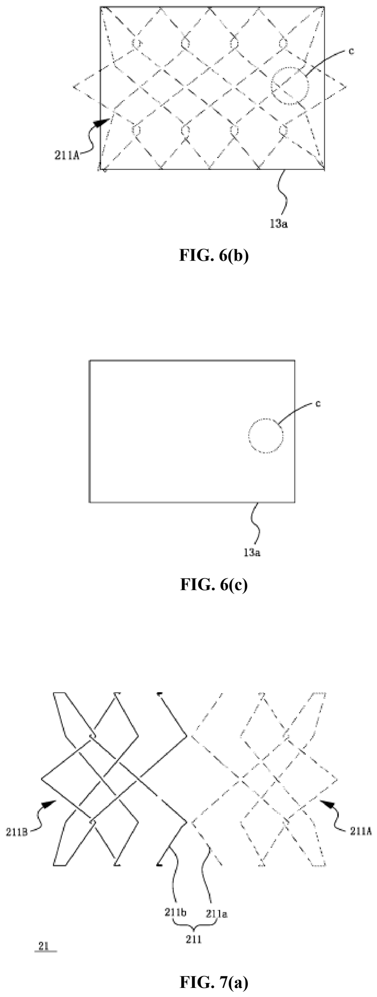

FIG. 6(a) is a schematic unfolded diagram of a main supporting layer, FIG. 6(b) is a schematic unfolded diagram of a connection between the main supporting layer in FIG. 6(a) and an edge of a recessed portion, and FIG. 6(c) is a schematic diagram of a main supporting layer in FIG. 6(b) after the degradation of a degradable layer.

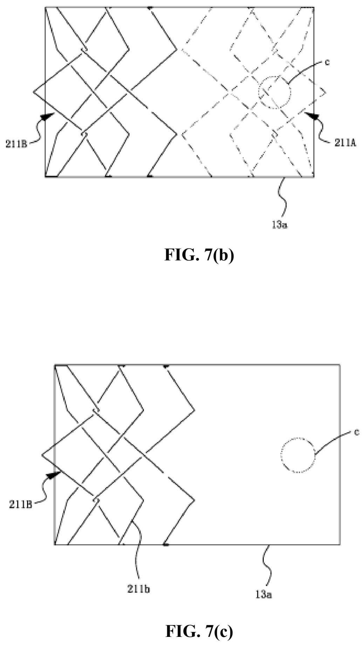

FIG. 7(a) is a schematic unfolded diagram of another main supporting layer, FIG. 7(b) is a schematic unfolded diagram of a connection between the main supporting layer in FIG. 7(a) and an edge of a recessed portion, and FIG. 7(c) is a schematic diagram of the main supporting layer in FIG. 7(b) after the degradation of a degradable layer.

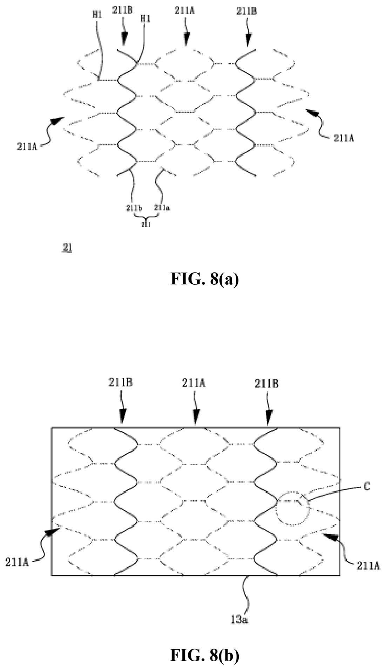

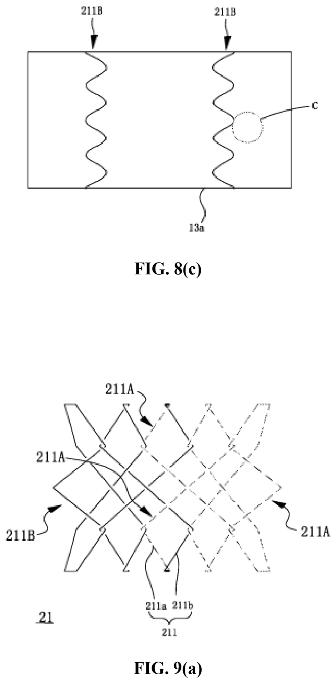

FIG. 8(a) is a schematic unfolded diagram of another main supporting layer, FIG. 8(b) is a schematic unfolded diagram of a connection between the main supporting layer in FIG. 8(a) and an edge of a recessed portion, and FIG. 8(c) is a schematic diagram of the main supporting layer in FIG. 8(b) after the degradation of a degradable layer.

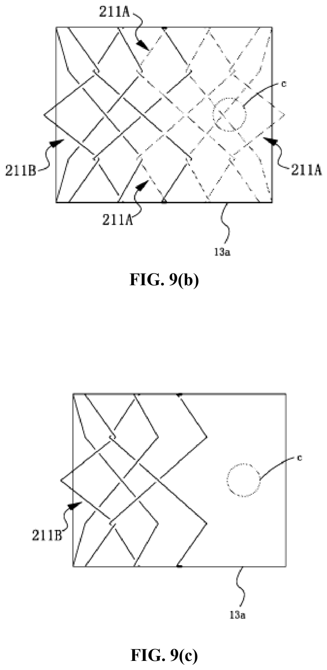

FIG. 9(a) is a schematic unfolded diagram of another main supporting layer, FIG. 9(b) is a schematic unfolded diagram of a connection between the main supporting layer in FIG. 9(a) and an edge of a recessed portion, and FIG. 9(c) is a schematic diagram of the main supporting layer in FIG. 9(b) after the degradation of a degradable layer.

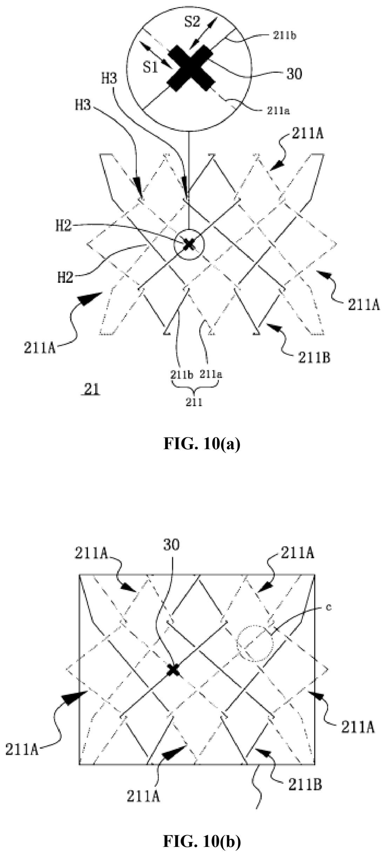

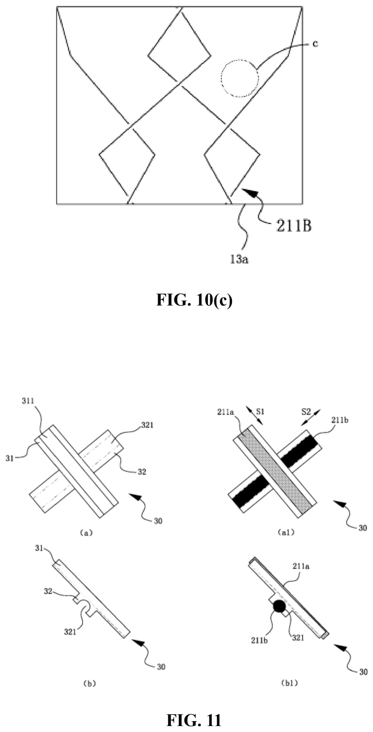

FIG. 10(a) is a schematic unfolded diagram of another main supporting layer provided with a fastener, FIG.10(b) is a schematic unfolded diagram of a connection between the main supporting layer in FIG. 10(a) and an edge of a recessed portion, and FIG. 10(c) is a schematic diagram of the main supporting layer in FIG. 10(b) after the degradation of a degradable layer.
FIG. 11(a) is a schematic top view of the fastener in FIG. 10(a), FIG. 11(b) is a schematic side view of the fastener in FIG. 10(a), FIG. 11(a1) is a schematic diagram of passage of supporting filaments through the fastener in FIG. 11(a), and FIG. 11(b1) is a schematic diagram of passage of supporting filaments through the fastener in FIG. 11(b).

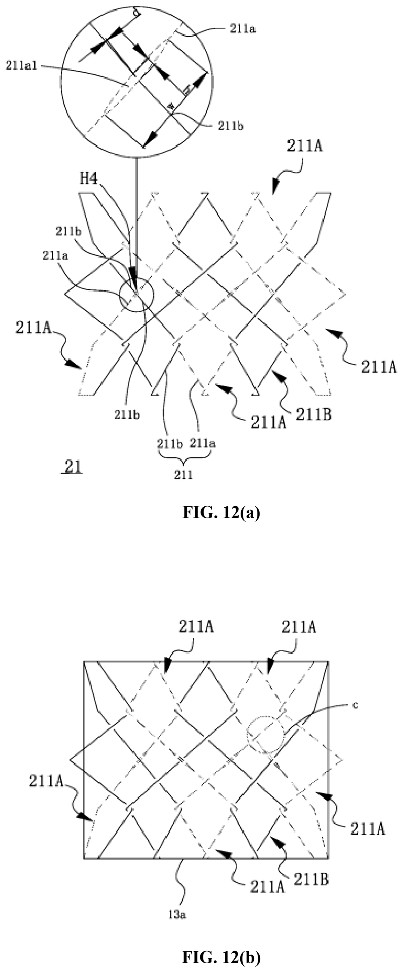

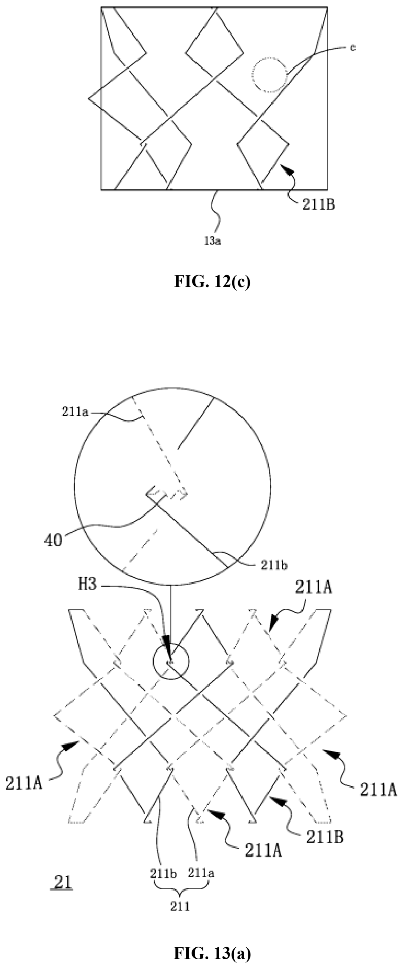

FIG. 12(a) is a schematic unfolded diagram of another main supporting layer, FIG. 12(b) is a schematic unfolded diagram of a connection between the main supporting layer in FIG. 12(a) and an edge of a recessed portion, and FIG. 12(c) is a schematic diagram of the main supporting layer in FIG. 12(b) after the degradation of a degradable layer.

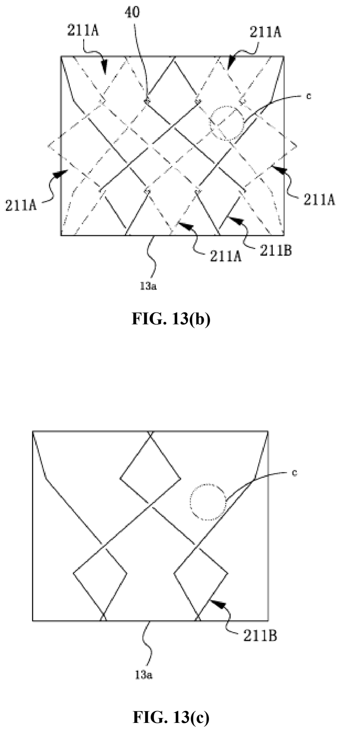

FIG. 13(a) is a schematic unfolded diagram of another main supporting layer provided with an elastic connecting member, FIG. 13(b) is a schematic unfolded diagram of a connection between the main supporting layer in FIG. 13(a) and an edge of a recessed portion, and FIG. 13(c) is a schematic diagram of the main supporting layer in FIG. 13(b) after the degradation of a degradable layer.

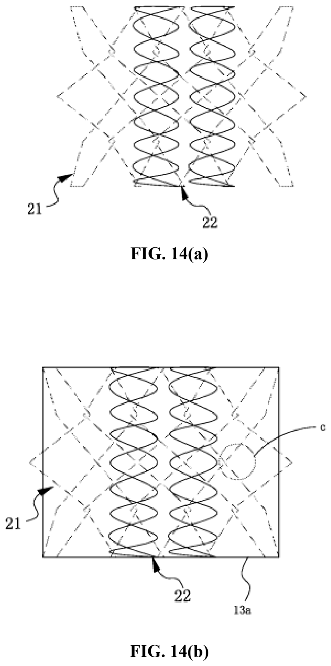

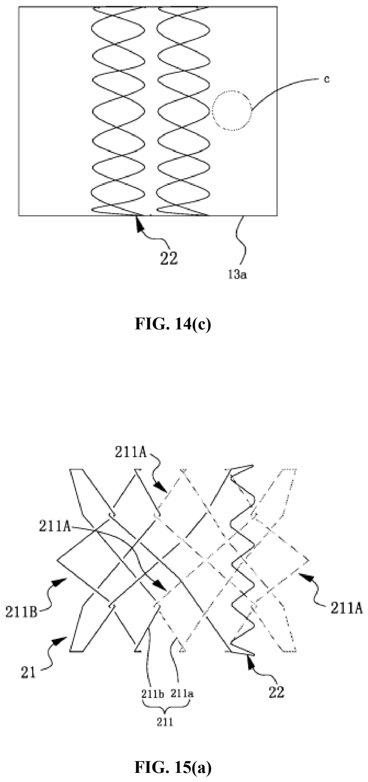

FIG. 14(a) is a schematic unfolded diagram of a main supporting layer and a local supporting layer, FIG. 14(b) is a schematic unfolded diagram of a connection between the main supporting layer and the local supporting layer in FIG. 14(a) and an edge of a recessed portion, and FIG. 14(c) is a schematic diagram of the main supporting layer and the local supporting layer in FIG. 14(b) after the degradation of a degradable layer within the main supporting layer.

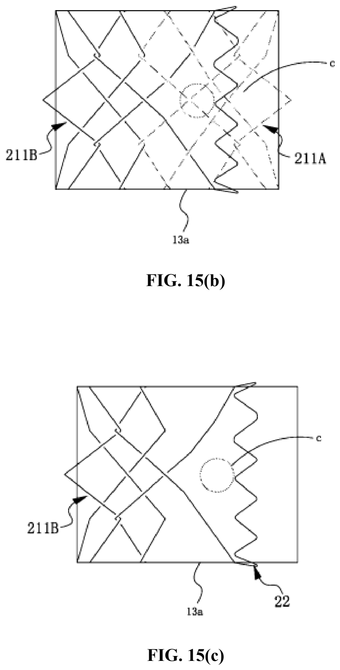

FIG. 15(a) is a schematic unfolded diagram of another main supporting layer and another local supporting layer, FIG. 15(b) is a schematic unfolded diagram of a connection between the main supporting layer and the local supporting layer in FIG. 15(a) and an edge of a recessed portion, and FIG. 15(c) is a schematic diagram of the main supporting layer and the local supporting layer in FIG. 15(b) after the degradation of a degradable layer within the main supporting layer.

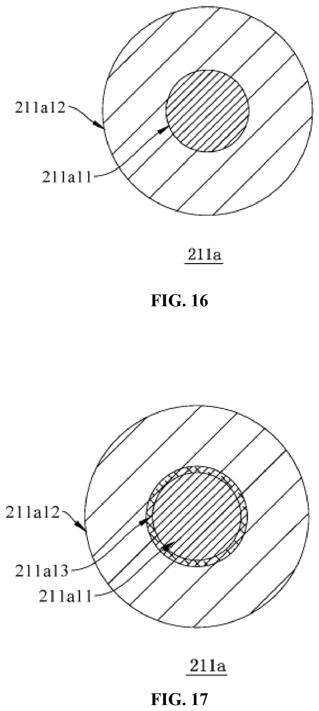

FIG. 16 is a schematic diagram of a multi-layer structure when a degradable first supporting filament is partially degraded.
FIG. 17 is schematic diagram of another multi-layer structure when a degradable first supporting filament is partially degraded.

### Detailed Description of the Invention

To make an objective, technical solutions, and advantages of the present invention more clearly, the exemplary implementations of the present disclosure will be described in more detail below with reference to the accompanying drawings. Although the accompanying drawings show the exemplary implementations of the present disclosure, it should be understood that the present disclosure may be implemented in various forms, and should not be limited to the implementations stated herein. Rather, these implementations are provided for understanding the present disclosure more thoroughly, and can completely transfer the scope of the present disclosure to those skilled in the art.

It should be understood that the terms used herein are only for the purpose of describing specific exemplary implementations and are not intended to be restrictive. Unless otherwise explicitly stated in the context, the singular forms such as "a/an", "one", and "the" used herein can also indicate a plural form. The terms such as "comprise", "include", "contain", and "have" are inclusive and specify the presence of stated features, steps, operations, elements, and /or components but do not exclude the presence or addition of one or more other features, steps, operations, elements, components, and /or combinations thereof. The method steps, processes, and operations described herein should not be construed as requiring execution in the specific sequence described or stated unless explicitly stated otherwise. It should be also understood that additional or alternative steps may be used.

Although the terms first, second, third, etc. may be used herein to describe multiple elements, components, areas, layers, and/or sections, these elements, components, areas, layers, and/or sections should not be limited by these terms. These terms can only be used to distinguish one element, component, area, layer, or section from another element, component, area, layer, or section. Unless explicitly stated otherwise, terms like "first" and "second" and other numerical terms do not imply a particular sequence or order. Thus, a first element, component, region, layer, or section discussed below may alternatively be referred to as a second element, component, region, layer, or section without departing from the teachings of the exemplary embodiments.

For ease of description, spatial relative relationship terms can be used herein to describe a relationship of an element or feature shown in the figure relative to another element or feature, such as "internal", "external", "inner side", "outer side", "beneath", "below", "on", and "above". This spatial relative relationship term means including different orientations of a device in use or operation, in addition to the orientations depicted in the figures. For example, if the device in the figure is flipped, then the elements described as "beneath other elements or features" or "below other elements or features" will subsequently be oriented as "on other elements or features" or "above other elements or features". Therefore, the exemplary term "below..." can include both above and below orientations. The device can be oriented in other ways (rotated 90 degrees or in other directions) and the spatial relative relationship descriptors used in the text are interpreted accordingly.

In order to describe the structure of the present application more clearly, the terms "proximal end" and "distal end" are defined herein as commonly used terms in the field of interventional medicine. Specifically, "distal end" means an end where blood flows out, and "proximal end" means an end where blood flows in. For example, after a stent is implanted, blood flows in from a proximal end of the stent and flows out from a distal end of the stent; and "axial direction" means a lengthwise direction of the stent, and "radial direction" means a direction perpendicular to the "axial direction".

Referring to FIG. 2, the present invention provides a vascular stent 100. The vascular stent 100 includes a main stent 10 provided with a main lumen 10a. The main stent 10 includes a proximal section 11, a distal section 12, and a middle section 13 located between the proximal section 11 and the distal section 12 and extending axially. The middle section 13 is provided with a recessed portion 131, above which a supporting structure 20 is arranged. At least part of the supporting structure 20 is degradable. To enhance supporting performance, the supporting structure 20 protrudes outward relative to the recessed portion 131. According to the vascular stent 100 of the present invention, at least part of the supporting structure 20 at the top of the vascular stent 100 is configured to be degradable. In this way, an initial mechanical supporting effect of a recess section is guaranteed while retaining the overall structure. In a long term, the material of this part can be automatically absorbed after a certain time, thereby reducing the restraint and compression on a branch stent, reducing the occupation of the main lumen space, and reducing irritation to vascular wall.

As shown in FIG. 2, the vascular stent 100 of the present invention includes the main stent 10 and the supporting structure 20. The main stent 10 has a hollow tubular structure with openings at both ends and is self-expandable, and the main lumen 10a is formed inside the hollow tubular structure. In other embodiments, the vascular stent 100 further includes an embedded branch 14 arranged in the main stent 10, and the main lumen 10a of the main stent 10 is communicated with a lumen of the embedded branch 14.

Exemplarily, the main stent 10 includes a main supporting portion and a main covering film. The main covering film may be arranged on an inner surface and/or an outer surface of the main supporting portion, or the main covering film may be arranged on part of an inner surface of the main supporting portion and the main covering film may be arranged on part of an outer surface of the main supporting portion. The main stent 10 may be axially divided into the proximal section 11, the distal section 12, and the middle section 13 located between the proximal section 11 and the distal section 12 and extending axially. The proximal section 11 includes a tubular proximal supporting portion and a proximal main covering film. The proximal main covering film may be applied to an inner side and/or an outer side of the proximal supporting portion by means of suturing, bonding, thermal fusion, or the like. The proximal supporting portion includes a plurality of main corrugated rings arranged axially at intervals. The distal section 12 includes a tubular distal supporting portion and a distal main covering film. The distal main covering film may be applied to an inner side and/or an outer side of the distal supporting portion by means of suturing, bonding, thermal fusion, or the like. The distal supporting portion includes a plurality of main corrugated rings arranged axially at intervals. The recessed portion 131 is arranged on the middle section 13, the supporting structure 20 is located above the recessed portion 131 of the middle section 13 and preferably protrudes outwards relative to the recessed portion 131. The middle section 13 includes a middle main covering film and a middle supporting portion (which may be omitted in other embodiments). A lumen enclosed by the middle main covering film is communicated with a lumen enclosed by the proximal main covering film and a lumen enclosed by the distal main covering film. At least part of an edge of the supporting structure 20 is fixedly connected to an edge 13a of the recessed portion by means of suturing, bonding, thermal fusion, or the like. A gap (also referred to as a void or cavity) is formed between the supporting structure 20 and an outer surface of the middle section 13, and may be communicated with the lumen of the embedded branch 14. At least part of the supporting structure 20 of the present invention is degradable, that is, the supporting structure 20 may be partially or entirely degradable. The term "degradable" refers to at least in vivo degradability, and a degradation rate may be controlled according to needs by adjusting a degradable material and a structure. According to the vascular stent 100 of the present invention, when a branch stent is needed to bridge a branch vessel, the branch stent may pass through the supporting structure 20 to be communicated with the embedded branch 14. During the reconstruction of the branch, the supporting structure 20 having supporting performance provides an initial mechanical supporting effect. Blood flow may enter the gap between the supporting structure 20 and the middle section 13 through the embedded branch 14, and then pass through the supporting structure 20 to enter the branch vessel, thereby avoiding prolonged ischemia in the branch vessel during reconstruction of the branch. For a twisted lumen, especially a lumen with a smaller true lumen, more stable operating space is provided during operation by arranging the gap between the supporting structure 20 and the middle section 13, thereby avoiding occupation of the operating space due to the compression of the lumen on a middle unit. After the branch stent is implanted, once at least part of the degradable supporting structure 20 is degraded and absorbed, a surrounding area is expanded. In this way, long-term compression on the branch stent can be reduced, and long-term irritation to a vascular wall and subsequent occupation of main lumen space can be reduced.

In the present invention, the "covering film" may isolate liquids to a certain extent and may be made from a polymer material with good biocompatibility, such as polytetrafluoroethylene (PTFE) and polyethylene terephthalate (PET).

As shown in FIG. 2, exemplarily, the supporting structure 20 is generally arc-shaped (in a naturally expanded state of the vascular stent 100), and a central angle corresponding to the projection of the supporting structure 20 on a radial plane is less than or equal to 120 degrees. In this way, the supporting structure 20 has a good radial supporting effect while ensuring sufficient space in the middle section 13 for blood flow; and at the same time, the middle section 13 maintains a relatively appropriate radial compression size.

To facilitate the passage of the branch stent through the supporting structure 20, a branch passage radially penetrating through the supporting structure 20 is arranged on the supporting structure 20. The branch passage allows the branch stent to pass through. At least part of an edge of the branch passage is degradable, so that the branch passage may be expanded upon at least partial degradation. The expanded branch passage may exert reduced compression on the branch stent passing through the expanded branch passage. Referring to FIG. 2 to FIG. 5, exemplarily, the supporting structure 20 may be a planar structure or a mesh structure. As shown in FIG. 3, the supporting structure 20 is a planar structure, and the branch passage may be a perforation a formed in the planar structure. Exemplarily, an outer peripheral region a1 of the perforation a is degradable. When the outer peripheral region a1 is degraded and absorbed, the perforation a is enlarged, thereby avoiding continuous compression on the branch stent passing through the perforation a. As shown in FIG. 2, FIG. 4, and FIG. 5, the supporting structure 20 is a mesh structure, grids b are formed on the mesh supporting structure 20 and may serve as the branch passage which allows the branch stent to pass through. Exemplarily, as shown in FIG. 4, at least part of the grids b serve as the branch passage which allows the branch stent to pass through. At least part of outer peripheral gratings b1 forming the grids b are degradable. When the outer peripheral gratings b1 are degraded and absorbed, the grids b are enlarged, thereby avoiding continuous compression on the branch stent passing through the grids b. It should be noted that when a plurality of grids exist, sizes and shapes of the grids can be set as needed. The grids may be same or different in shapes and sizes. One or more of the grids may be selected to serve as the branch passage. As shown in FIG. 5, during manufacturing, the prefabricated supporting structure 20 is assembled with the main stent 10. During assembly, an outer peripheral edge of the supporting structure 20 may be fastened relative to the edge 13a of the recessed portion 131. The fastening manner includes, but is not limited to, suturing the outer peripheral edge of the supporting structure to the covering film located at the edge 13a. Once fastened, the supporting structure 20 protrudes outwards relative to the recessed portion and is generally arc-shaped.

To facilitate communication between the embedded branch 14 located at a lateral side of the recessed portion 131 and the branch vessel, the branch passage is typically arranged adjacent to a lateral side of the supporting structure 20 in an axial direction. For example, when one or two embedded branches 14 are connected to a proximal side of the main stent 10, the branch passage may be arranged near a proximal side of the supporting structure 20, and in this case, one or two branch passages may be arranged near the proximal side. When one or two embedded branches 14 are connected to a distal side of the main stent 10, the branch passage may be arranged near a distal side of the supporting structure 20, and in this case, one or two branch passages may be arranged near the distal side. When the embedded branches are arranged on both the proximal side and distal side of the main stent 10, the branch passages may be arranged near both the proximal side and distal side of the supporting structure 20. As shown in FIG. 2, in this embodiment, two embedded branches 14 are connected to the proximal side of the main stent 10, and one embedded branch 14 is connected to the distal side. Two branch passages are arranged near the proximal side of the supporting structure 20, one branch passage is arranged near the distal side, and each branch passage corresponds to a respective embedded branch.

Referring to FIG. 2 to FIG. 13, the supporting structure 20 includes a main supporting layer 21 that is at least partially degradable. When the main supporting layer 21 is of a mesh structure, an implementation of the main supporting layer 21 having the mesh structure of the present invention is illustrated by a mesh shape shown in FIG. 6(a). In an unfolded or expanded state (i.e., a natural state of the main supporting layer 21 prior to being connected to the main stent 10), the main supporting layer 21 includes, in a circumferential direction, a first mesh region and a second mesh region connected to the first mesh region. The first mesh region includes a plurality of rows of intersection units which are formed by mutually overlapping a plurality of first-direction supporting filaments arranged at intervals and a plurality of second-direction supporting filaments arranged at intervals. The second mesh region includes at least one row of hooking units, and each row of hooking units includes one or more axially arranged hooking units. Each hooking unit includes a first hooking member and a second hooking member. In at least part of the hooking units, the first hooking member and the second hooking member are substantially hooked with each other in an axial direction. Alternatively, in at least part of the hooking units, the first hooking member and the second hooking member are substantially separated from each other in an axial direction to form a gap. Alternatively, in at least part of the hooking units, the first hooking member and the second hooking member are abutted against each other in a non-hooking manner.

The main supporting layer 21 of the mesh structure is formed by supporting filaments 211, and the supporting filaments 211 include at least a degradable first supporting filament 211a.

For clarity of description, in the labeling of the drawings of the present invention, dashed lines represent degradable components, while solid lines represent non-degradable components. Referring to FIG. 6(a) to FIG. 6(c), in one embodiment, the supporting filaments 211 include only the degradable first supporting filaments 211a. The degradable first supporting filaments 211a form the main supporting layer 21, i.e., the main supporting layer 21 is entirely formed by the degradable first supporting filaments 211a and has a single-layer structure. As such, the main supporting layer 21 is degradable. At least part of an edge of the main supporting layer 21 formed by the degradable first supporting filaments 211a is fastened relative to the edge 13a of the recessed portion. After the main supporting layer 21 is degraded and absorbed, the first supporting filaments 211a located inside the edge 13a of the recessed portion are degraded, such that an opening c corresponding to the embedded branch 14 is no longer obstructed. As a result, when connected to the embedded branch 14, the branch stent is no longer be restrained or compressed by the supporting filaments.

Referring to FIG. 7(a) to FIG. 15(c), in another embodiment, the supporting filaments 211 include not only the degradable first supporting filament 211a but also a non-degradable second supporting filament 211b, that is, the degradable first supporting filament 211a and the non-degradable second supporting filament 211b jointly form the main supporting layer 21. In other words, the main supporting layer 21 in this embodiment is not entirely degradable, but partially degradable. The partial degradation helps relieve restraint and compression, while the non-degradable portion provides sustained support.

The degradable first supporting filament 211a of the present invention is either entirely degradable or partially degradable. Referring to FIG. 16 and FIG. 17, when the first supporting filament 211a is partially degradable, the first supporting filament 211a has a multi-layer structure arranged from inside to outside, and includes a non-degradable inner core 211a11 located at an innermost layer and a degradable covering layer 211a12 located at an outermost layer. The inner core 211a11 is non-degradable and made from a non-degradable material, while the covering layer 211a12 is degradable and made from a degradable material. Exemplarily, the degradable covering layer 211a12 may be coated onto the inner core 211a11. Alternatively, in other embodiments, as shown in FIG. 17, the first supporting filament 211a further includes an adhesive layer 211a13 arranged between the inner core 211a11 and the covering layer 211a12, whereby the covering layer 211a12 is fixedly arranged on the inner core 211a11 through the adhesive layer 211a13. According to the multi-layer structure of the degradable first supporting filament 211a in this embodiment, the non-degradable filament is taken as the inner core, thereby greatly improving radial supporting performance of a mesh cover. Additionally, a radial supporting strength is uniformly distributed, thereby minimizing damage to vascular wall. After the outer covering layer is degraded and absorbed, a diameter of the supporting corrugated ring filament is reduced, thereby achieving a therapeutic effect of alleviating compression on an arch branch stent and vascular wall. The degradable first supporting filament 211a and the non-degradable second supporting filament 211b jointly form the main supporting layer 21 by at least the following implementation manners.

Implementation manner 1: referring to FIG. 7(a) to FIG. 7(c), the first supporting filament 211a forms a degradable layer 211A, and the second supporting filament 211b forms a non-degradable layer 211B. The degradable layer 211A and the non-degradable layer 211B are arranged opposite to each other within a same layer, where "opposite" may refer to that the degradable layer and the non-degradable layer are spaced apart and opposite to each other, or the degradable layer and the non-degradable layer are abutted against and opposite to each other. The degradable layer 211A formed by the first supporting filament 211a and the non-degradable layer 211B formed by the second supporting filament 211b are relatively independent. It should be understood that relative distribution regions of the layers can be arranged as needed, and the illustrations are provided for exemplary purposes only. In this manner, the degradable layer 211A and the non-degradable layer 211B may be respectively fastened to the main stent 10 to form the main supporting layer 21, or may first be fastened to each other via a connecting member and then collectively fastened to the main stent 10. It should be noted that the fastening manner is not limited to the examples mentioned. Exemplarily, edges of both the degradable layer 211A (formed by the degradable first supporting filament 211a) and the non-degradable layer 211B (formed by the non-degradable second supporting filament 211b) are at least partially fastened relative to the edge 13a of the recessed portion. After the degradable layer 211A is degraded and absorbed, the opening c corresponding to the embedded branch 14 is no longer obstructed, so that when connected to the embedded branch, the branch stent is no longer be restrained or compressed by the supporting filament.

Implementation manner 2: referring to FIG. 8(a) to FIG. 8(c), the first supporting filament 211a forms a degradable layer 211A, and the second supporting filament 211b forms a non-degradable layer 211B. The degradable layer 211A and the non-degradable layer 211B are adjacent to and connected to each other at an adjacent position. That is, the degradable layer 211A formed by the first supporting filament 211a and the non-degradable layer 211B formed by the second supporting filament 211b are adjacent and connected with each other. This connection may be direct connection, as shown in FIG. 8(a), or may be connection through other connecting members (not shown). In this embodiment, since the two layers are relatively fastened, edges of both the degradable layer 211A (formed by the degradable first supporting filament 211a) and the non-degradable layer 211B (formed by the non-degradable second supporting filament 211b) are at least partially fastened relative to the edge 13a of the recessed portion. After the degradable layer 211A is degraded and absorbed, the opening c corresponding to the embedded branch 14 is no longer obstructed, so that when connected to the embedded branch, the branch stent is no longer be restrained or compressed by the supporting filament.

Implementation manner 3: referring to FIG. 9(a), FIG. 10(a), FIG. 12(a), and FIG. 13(a), the first supporting filament 211a and the second supporting filament 211b are arranged in an interlaced manner within a same layer, such that the first supporting filament 211a forms a plurality of degradable layers 211A, and the second supporting filament 211b forms a non-degradable layer 211B. The plurality of degradable layers 211A are distributed in the non-degradable layer 211B within the same layer, i.e., the degradable layers 211A formed by the first supporting filament 211a are not confined to one location but are distributed in a plurality of regions. In this manner, not only regions where the degradable layers 211A are located can be flexibly configured as needed, but also the overall supporting performance of the supporting structure 20 can be ensured as much as possible. In this embodiment, since the two layers are relatively fastened, the edges of both the degradable layers 211A (formed by the degradable first supporting filament 211a) and the non-degradable layer 211B (formed by the non-degradable second supporting filament 211b) may be at least partially fastened relative to the edge 13a of the recessed portion. After the degradable layer 211A is degraded and absorbed, the opening c corresponding to the embedded branch 14 is no longer obstructed, so that when connected to the embedded branch, the branch stent is no longer be restrained or compressed by the supporting filament. It should be noted that the term "distributed" in this embodiment is defined in relation to the regions where the respective components are located and does not necessarily imply that the plurality of degradable layers 211A are completely independent from one another. The plurality of distributed degradable layers can either be independent or interconnected. Exemplarily, if a single first supporting filament 211a is arranged in an interlaced manner (such as interlaced braiding), the distributed degradable layers 211A are actually connected to each other.

Additionally, it should be noted that the term "same layer" in the present invention does not limit the supporting filaments 211 forming the supporting structure to be a single layer. During the configuration process, as needed, the supporting filaments 211 may also be stacked or hooked, etc. In the design, one, two, or more of the abovementioned manners may be selected as needed. These manners can be freely combined, as long as they do not conflict with each other, and such combinations also fall within the scope of the protection of the present invention.

Referring to FIG. 9(a) to FIG. 9(c), the first supporting filament 211a forms the degradable layer 211A, and the second supporting filament 211b forms the non-degradable layer 211B. The degradable layer 211A and the non-degradable layer 211B are connected in an interlaced manner at respectively opposite edges. That is, the degradable layer 211A formed by the first supporting filament 211a and the non-degradable layer 211B formed by the second supporting filament 211b are interlaced and distributed at the edges. This interlaced connection at the edges ensures both reliability of connection and required supporting performance. In this embodiment, since the two layers are relatively fastened, the edges of both the degradable layer 211A (formed by the degradable first supporting filament 211a) and the non-degradable layer 211B (formed by the non-degradable second supporting filament 211b) are at least partially fastened relative to the edge 13a of the recessed portion. After the degradable layer 211A is degraded and absorbed, the opening c corresponding to the embedded branch 14 is no longer obstructed, so that when connected to the embedded branch, the branch stent is no longer be restrained or compressed by the supporting filament.

In order to ensure an initial supporting effect of the vascular stent 100, during the formation of the main supporting layer 21, a plurality of supporting filaments 211 are required to be intersected to form intersection points; and at the intersection points, the plurality of supporting filaments 211 are interconnected, and/or overlapped, and/or hooked. That is, the supporting filaments 211 at the intersection points may be connected by one or more of connection, overlapping, and hooking. As shown in FIG. 8(a), the supporting filaments at an intersection point H1 are directly connected; as shown in FIG. 10(a), the supporting filaments at an intersection point H2 are arranged in an overlapped manner (similar to a cross); and the supporting filaments at an intersection point H3 are connected in a hooking manner. The manners of intersection between the supporting filaments at the intersection points may be freely selected.

Since the first supporting filament 211 is degradable, supporting performance is relatively low. During the self-expansion of the vascular stent 100, it is crucial that the first supporting filament 211 is fully expanded in order to ensure the formation of the recess space in initial implantation. In view of this, the present invention proposes the following further solutions.

In one embodiment, as shown in FIG. 10(a) to FIG. 10(c), when the plurality of supporting filaments 211 are overlapped at the intersection points, the supporting filaments 211 at the intersection points may be connected via a fastener 30, and the fastener 30 is configured to radially restrain the supporting filaments 211 to avoid radial separation from one another. For example, as shown in FIG. 10(a), two supporting filaments 211 are arranged at the intersection point H2. Of course, the supporting filaments 211 at this point may all be the degradable first supporting filaments 211a, or may include both the degradable first supporting filament 211a and the non-degradable second supporting filament 211b. The fastener 30 connects the two supporting filaments 211, ensuring that during the compression and subsequent self-expansion of the vascular stent 100, the two supporting filaments 211 expand together, avoiding the problem that the degradable first supporting filament 211a, due to poor elasticity, is difficult to expand. This effectively ensures the initial supporting performance of the vascular stent 100. In this embodiment, the fastener 30 is degradable. In this embodiment, since the two layers are relatively fastened, the edges of both the degradable layer 211A (formed by the degradable first supporting filament 211a) and the non-degradable layer 211B (formed by the non-degradable second supporting filament 211b) may be at least partially fastened relative to the edge 13a of the recessed portion. After the degradable layer 211A and the fastener 30 are degraded and absorbed, the opening c corresponding to the embedded branch 14 is no longer obstructed, so that when connected to the embedded branch, the branch stent is no longer be restrained or compressed by the supporting filament.

Further, since the vascular stent 100 needs to be compressed into a delivery device by an external force before implantation, and is expected to exhibit good self-expansion after release, referring to FIG. 10(a) and FIG. 11, when the supporting filaments 211 at the intersection points are connected with each other via the fastener 30, a first sliding passage 31 extending in a first predetermined direction S1 and a second sliding passage 32 extending in a second predetermined direction S2 are formed on the fastener 30; one supporting filament 211a may slide relative to the fastener 30 along the first predetermined direction S1, while the other supporting filament 211b may slide relative to the fastener 30 along the second predetermined direction S2, and the first predetermined direction S1 and the second predetermined direction S2 are predetermined movement directions of the supporting filaments 211 when the vascular stent 100 is compressed or self-expands; typically, the first predetermined direction S1 and the second predetermined direction S2 are extension directions of the supporting filaments 211 at the intersection points. For example, as shown in FIG. 10(a) and FIG. 11, the first supporting filament 211a slides along the first predetermined direction S1 in the first sliding passage 31, and the second supporting filament 211b slides along the second predetermined direction S2 in the second sliding passage 32. However, the fastener 30 always radially restrains the first supporting filament 211a and the second supporting filament 211b together. When the vascular stent 100 is compressed or self-expands, the two supporting filaments 211 connected to the fastener 30 expand together, and in this way, the first supporting filament 211a expands as the second supporting filament 211b expands.

In another embodiment, when the plurality of supporting filaments 211 include a first supporting filament 211a and a second supporting filament 211b, and the first supporting filament 211a and the second supporting filament 211b are overlapped at intersection points, in order to ensure effective expansion of the first supporting filament 211a, the first supporting filament 211a is located above the second supporting filament 211b at the intersection points. During the expansion, the second supporting filament 211b, which has relatively good self-expanding performance, can support the first supporting filament 211a located above the second supporting filament. In this way, the first supporting filament 211a can smoothly expand and provide support; and occupation of the internal operating space caused by incomplete expansion of the first support filament 211a can be avoided. In this way, since the first supporting filament 211a is located above the self-expanding second supporting filament 211b, the first supporting filament 211a can be expanded with the support of the second supporting filament 211b even without a fastener 30.

In another embodiment, referring to FIG. 12(a) to FIG. 12(c), when the plurality of supporting filaments 211 includes a first supporting filament 211a and a second supporting filament 211b, and the first supporting filament 211a and the second supporting filament 211b are overlapped at an intersection point (e.g., at an intersection point H4 shown in FIG. 12(a)), a radial thickness h of the first supporting filament 211a at least at the intersection point is greater than a radial thickness d of the second supporting filament 211b (d corresponds to the diameter if the second supporting filament 211b is cylindrical). Furthermore, a cross passage 211a1 through which the second supporting filament 211b passes is formed at the intersection point of the first supporting filament 211a. In this embodiment, as shown in FIG.12(a), at the intersection point H4, the second supporting filament 211b passes through the first supporting filament 211a, such that the self-expandable second supporting filament 211b is at least partially covered by the first supporting filament 211a. During the expansion of the first supporting filament 211a, the self-expandable second supporting filament 211b provides outward support to the first supporting filament 211a, whereby the first supporting filament 211a can smoothly expand and provide support. In addition, another advantage of this embodiment is that if the degradable layer 211A formed by the first supporting filament 211a is formed by 3D printing or injection molding, the formation of the cross passage 211a1, through which the second supporting filament 211b passes, of the first supporting filament 211a can be easily implemented, and a process is relatively simple. For example, the second supporting filament 211b may be braided first, and then the first supporting filament 211a is formed by 3D printing or injection molding, such that the second supporting filament 211b is covered in the first supporting filament at the intersection point. Alternatively, during the 3D printing or injection molding of the first supporting filament 211a, the cross passage 211a1 is reserved, and then the second supporting filament 211b is threaded through the cross passage 211a1 when braided. Further, in this embodiment, to avoid an excessive thickness of the first supporting filament 211a at the intersection point, the cross passage 211a1 may be arranged in a semi-arc shape and is only required to partially cover the second supporting filament 211b. In this embodiment, the first supporting filament 211a and the second supporting filament 211b are arranged in an interlaced manner within a same layer, a plurality of degradable layers 211A are distributed in the non-degradable layer 211B within the same layer, the edges of both the degradable layer 211A (formed by the degradable first supporting filament 211a) and the non-degradable layer 211B (formed by the non-degradable second supporting filament 211b) are at least partially fastened relative to the edge 13a of the recessed portion. After the degradable layer 211A is degraded and absorbed, the opening c corresponding to the embedded branch 14 is no longer obstructed, so that when connected to the embedded branch, the branch stent is no longer be restrained or compressed by the supporting filament.

Referring to FIG. 12(a) again, when the cross passage 211a1 through which the second supporting filament 211b passes is arranged on the first supporting filament 211a, the cross passage 211a1 has a predetermined length w along an extension direction of the first supporting filament 211a. The predetermined length w is configured to ensure that when the vascular stent 100 is compressed or self-expands, the second supporting filament 211b has certain movement space, thereby facilitating the smooth compression and expansion of the supporting structure 20, and providing effective and stable support.

In another embodiment, referring to FIG. 13(a) to FIG.13 (c), when the plurality of supporting filaments are arranged in a hooking manner, as shown in FIG. 13(a), at the intersection point H3, an elastic connecting member 40 which axially extends is arranged between the supporting filaments 211 at a hooking position. End portions of the elastic connecting member 40 are respectively connected to hooked portions of the plurality of supporting filaments 211. The elastic connecting member 40 is configured to elastically pull the supporting filaments 211 toward each other. Similarly, the elastic connecting member 40 provides good connectivity, ensures that the supporting filaments 211 are radially compressed or expanded simultaneously as the vascular stent 100 is compressed or self-expands, and allows the supporting filaments 211 connected to the elastic connecting member to have certain movement space. In this embodiment, the elastic connecting member 40 is degradable. In this embodiment, the first supporting filament 211a and the second supporting filament 211b are arranged in an interlaced manner within a same layer, the plurality of degradable layers 211A are distributed in the non-degradable layer 211B within the same layer, and the edges of both the degradable layer 211A (formed by the degradable first supporting filament 211a) and the non-degradable layer 211B (formed by the non-degradable second supporting filament 211b) are at least partially fastened relative to the edge 13a of the recessed portion. After the degradable layer 211A and the elastic connecting member 40 are degraded and absorbed, the opening c corresponding to the embedded branch 14 is no longer obstructed, so that when connected to the embedded branch, the branch stent is no longer be restrained or compressed by the supporting filament.

The degradable first supporting filament 211a of the present invention may be formed by 3D printing, and/or injection molding, and/or metal cutting, and/or braiding. The non-degradable second supporting filament 211b may be formed by metal cutting and/or braiding. It should be understood that one or more of the aforementioned formation methods may be adopted. Of course, it is not limited thereto.

Referring to FIG. 14(a) and FIG. 15(a), in order to further ensure supporting performance of the partially degradable supporting structure 20, the supporting structure 20 further includes a non-degradable local supporting layer 22, that is, the supporting structure 20 includes a main supporting layer 21 and a local supporting layer 22. The local supporting layer 22 and the main supporting layer 21 are stacked from inside to outside, that is, the main supporting layer 21 is located outside and the local supporting layer 22 is located inside. The main supporting layer 21 is located above the local supporting layer 22, and the local supporting layer 22 is opposite to the degradable layer 211A formed by the first supporting filament 211a in the main supporting layer 21. The inner-outer dual-layer arrangement allows the local supporting layer 22 located inside to effectively support the degradable layer 211A located outside. At the same time, this arrangement enhances the flexibility of the supporting structure 20, lowers requirements for the manufacturing process, and increases efficiency.

Referring to FIG. 14(a) to FIG. 14(c), the supporting structure 20 includes a main supporting layer 21 and a local supporting layer 22, the main supporting layer 21 is entirely formed by a degradable first supporting filament 211a and located outside; the local supporting layer 22 is formed by a non-degradable elastic corrugated ring, the local supporting layer 22 is arranged near a middle portion on an inner layer of the main supporting layer 21, and edges of both a degradable layer 211A (formed by the degradable first supporting filament 211a) and the local supporting layer 22 are at least partially fastened relative to the edge 13a of the recessed portion. After the degradable layer 211A located outside is degraded and absorbed, the opening c corresponding to the embedded branch 14 is no longer obstructed, so that when connected to the embedded branch, the branch stent is no longer be restrained or compressed by the supporting filament. The local supporting layer 22 is non-degradable and continuously provides support.

Referring to FIG. 15(a) to FIG. 15(c), the supporting structure 20 includes a main supporting layer 21 and a local supporting layer 22, the main supporting layer 21 includes a degradable first supporting filament 211a and the non-degradable second supporting filament 211b; the first supporting filament 211a forms a degradable layer 211A, the second supporting filament 211b forms a non-degradable layer 211B, the degradable layer 211A and the non-degradable layer 211B are connected in an interlaced manner at respectively opposite edges. That is, the degradable layer 211A formed by the first supporting filament 211a and the non-degradable layer 211B formed by the second supporting filament 211b are interlaced and distributed at the edges. The local supporting layer 22 is formed by a non-degradable elastic corrugated ring. The local supporting layer 22 is arranged near the distal side and located on an inner layer of the main supporting layer 21. Edges of both the degradable layer 211A (formed by the degradable first supporting filament 211a) and the non-degradable layer 211B (formed by the non-degradable second supporting filament 211b), and at least part of an edge of the local supporting layer 22 are fastened relative to the edge 13a of the recessed portion. After the degradable layer 211A located outside is degraded and absorbed, the opening c corresponding to the embedded branch 14 is no longer obstructed, so that when connected to the embedded branch, the branch stent is no longer be restrained or compressed by the supporting filament. The local supporting layer 22 is non-degradable and continuously provides support.

The degradable material of the present invention includes a degradable polymer material and/or a degradable metal material.

Exemplarily, the degradable polymer material includes at least one of polylactic acid, polyglycolic acid, polybutylene succinate, poly(β-hydroxybutyrate), poly(β-alkanoate), polycaprolactone, poly(ethylene adipate), polylactic-co-glycolic acid, polyhydroxybutyrate-co-hydroxyvalerate, polyvinyl alcohol, polyethylene glycol, polyvinylpyrrolidone, starch, cellulose, polyethylene oxide, pectin, collagen, chitosan, dextran, chitin, and derivatives of the above polymers; or a copolymer formed by copolymerization of at least two monomers that constitute the aforementioned materials. The degradable metal material includes at least one of zinc, magnesium, iron, a zinc-based alloy, a magnesium-based alloy, and an iron-based alloy. It should be noted that the degradable material is not limited to the above-mentioned.

According to the vascular stent of the present invention, at least part of the supporting structure at the top of the vascular stent is configured to be degradable. In this way, the initial mechanical supporting effect of a recess section is guaranteed while retaining the overall structure. In a long term, the material of this part can be automatically absorbed after a certain time, thereby reducing the restraint and compression on a branch stent, reducing the occupation of the main lumen space, and reducing irritation to vascular wall.

Based on the overall structure of the vascular stent 100 described above, a brief exemplary description of the structure of the present invention is made with reference to specific drawings and embodiments. For the structure of the main stent 10 of the vascular stent 100, refer to the foregoing descriptions. Details are not described again below. The following embodiments will primarily illustrate the specific implementation of the supporting structure 20. It should be noted that the various embodiments may be selectively combined with one another.

### Embodiment 1

Referring to FIG. 6(a) to FIG. 6(c), in this embodiment, a supporting structure 20 is a mesh structure. The mesh supporting structure 20 is formed by a degradable first supporting filament 211a. That is, a main supporting layer 21 is entirely formed by the degradable first supporting filament 211a (a dashed line represents the degradable first supporting filaments 211a) and has a single-layer structure. The main supporting layer 21 is degradable. The degradable supporting structure in this embodiment may be made of a biocompatible absorbable material. The main supporting layer 21 is absorbable in vivo in a long term, with a controllable degradation rate. It exhibits excellent machinability, mechanical strength, and a controllable degradation rate.

The supporting structure has a certain radial supporting strength to support recess space and facilitate selection using an internal branch guidewire, and has good flexibility to reduce irritation and damage to vascular wall. Compared with the conventional vascular stent, the main supporting layer 21 can be completely absorbed; and in this way, long-term compression on a branch stent can be reduced, and long-term irritation to the vascular wall and subsequent occupation of main lumen space can be reduced.

### Embodiment 2

Referring to FIG. 8(a) to FIG. 8(c), in this embodiment, a supporting structure 20 is a mesh structure (different from the mesh structure in Embodiment 1). In Embodiment 2, the supporting structure is a spaced degradable structure. The mesh supporting structure 20 in Embodiment 2 is interlaced from a degradable first supporting filament 211a and a non-degradable second supporting filament 211b (a dashed line represents the degradable first supporting filament 211a, and a solid line represents the non-degradable second supporting filament 211b).

In this embodiment, a supporting corrugated ring braided from the non-degradable second supporting filament made of a memory metal alloy provides better radial supporting strength compared to a supporting corrugated ring braided from the degradable first supporting filament. If the supporting strength of the supporting corrugated ring is insufficient, it may lead to inadequate recess space, especially in an area adjacent to an embedded branch. Insufficient radial supporting strength may affect the selection using an internal branch guidewire and increase the difficulty of an operation. In this embodiment, the structure formed by braiding the degradable and non-degradable materials with space is adopted and better supporting performance can be achieved by use of mechanical properties of the non-degradable material compared to Embodiment 1. Additionally, in this embodiment, part of the structure is made of the degradable material, so as to reduce a long-term existing area of the supporting corrugated ring and effectively reduce irritation to vascular wall and downward compression and occupation on a main lumen. Upon degradation, restraint and compression on a branch stent are reduced.

### Embodiment 3

Referring to FIG. 9(a) to 9(c), this embodiment has a structure similar to the mesh structure in Embodiment 1, and a difference is that in this embodiment, a main supporting layer 21 is interlaced from a degradable first supporting filament 211a and a non-degradable second supporting filament 211b and adopts a degradable and non-degradable junction structure. A number of degradable units is determined according to a proportion of the degradable portion, which is set by an actual size of the aorta of a patient. The degradable portion indicated by a dashed line is adjacent to the left subclavian artery. The dashed line represents the degradable first supporting filament 211a, and a solid line represents the non-degradable second supporting filament 211b. A crimping pattern is shown in the figure and a break line indicates that the respective filament is located below. When the first supporting filament 211a and the second supporting filament 211b are overlapped at an intersection point, the degradable first supporting filament 211a at the intersection point is located above the non-degradable second supporting filament 211b. Additionally, crimping directions at two adjacent intersection points are opposite to ensure the stability of the structure and form. This embodiment ensures a radial supporting effect of a supporting corrugated ring, and the supporting corrugated rings adjacent to a left subclavian artery stent are degradable, thereby effectively or completely alleviating a negative compressive effect on the branch stent.

### Embodiment 4

Referring to FIG. 10(a) to 10(c), this embodiment has a structure similar to the mesh structures in Embodiments 1 and 3, and a difference is that this embodiment adopts a spaced absorbable structure. The remaining structure and operating principle are the same as those in Embodiment 1. After an absorbable layer in Embodiment 3 is absorbed, a right-side tip portion is susceptible to an external puncture force (shown in FIG. 9(c)), which may cause damage to vascular wall. However, this embodiment does not have a monofilament tip structure after degradation (shown in FIG. 10(c)), thereby preventing damage. This embodiment not only effectively avoids the compression after the implantation of a left subclavian artery branch stent, but also offers better radial supporting performance and more uniform radial supporting strength. The proportion of non-absorbable units is reduced.

In addition, to enhance the supporting performance, a degradable first supporting filament 211a is located above a non-degradable first supporting filament, and a cross-shaped fastener 30 is added to improve the structural stability.

### Embodiment 5

Referring to FIG. 13(a) to 13(c), this embodiment has a structure similar to the mesh structures in Embodiments 1, 3, and 4, and a difference is that an elastic connecting member 40 is added at a hooking position to enhance the supporting performance. When the supporting corrugated ring is in a horizontal state, the elastic connecting member 40 is in a tensioned state. After the stent is released, the supporting corrugated ring corresponds to an aortic arch, and the supporting corrugated ring bends as the stent self-expands after implantation. The elastic connecting member 40, in order to restore a natural state, pulls filaments on both sides, thereby reducing hooking spacing between the filaments and strengthening a supporting effect of the supporting corrugated ring.

### Embodiment 6

Referring to FIG. 12(a) to 12(c), this embodiment has a structure similar to the mesh structures in Embodiments 1, 3, 4, and 5, and a difference is that this embodiment is based on Embodiment 4 and adopts a structure shown in FIG. 12(a). Considering that a brachiocephalic trunk stent and a left common carotid artery stent are less prone to compression and deformation by a supporting corrugated ring compared to a left subclavian artery stent, but the supporting corrugated ring in this location needs sufficient radial supporting strength to support the vascular wall and maintain recess space, this embodiment retains the use of conventional non-absorbable materials for portions adjacent to the brachiocephalic trunk and the left common carotid artery. Since space adjacent to the brachiocephalic trunk/left common carotid artery is required to be sufficient to facilitate the selection of the brachiocephalic trunk and the left common carotid artery. This imposes high requirements for supporting performance of the material. Therefore, in this embodiment, a non-absorbable material is adopted to maintain the original radial supporting performance and ensure adequate space for branch stent selection. A number of required stents and a proportion of absorbable units can be adjusted based on an actual condition of the aorta of a patient.

### Embodiment 7

Referring to FIG. 14(a) to 14(c), this embodiment adopts a double-layer structure, that is, a local supporting layer 22 is added. A supporting structure 20 includes a main supporting layer 21 and a local supporting layer 22. The local supporting layer 22 and the main supporting layer 21 are stacked from inside to outside, i.e., the main supporting layer 21 is located outside, the local supporting layer 22 is located inside, and the main supporting layer 21 is located above the local supporting layer 22. The local supporting layer 22 includes two corrugated rings, which are placed beneath the absorbable main supporting layer 21. The local supporting layer is located in the middle of the absorbable main supporting layer 21 and does not interfere with the implantation of a branch stent. The double-layer design enhances the radial supporting performance of the absorbable portion, and a proportion of absorbable units is high. After absorption of the absorbable units, only the two corrugated rings remain, which is beneficial for vascular endothelial recovery. Furthermore, a radial supporting strength is evenly distributed, minimizing the risk of damage to the vascular wall.

### Embodiment 8

Referring to FIG. 8(a) to 8(c), in this embodiment, a first supporting filament 211a forms a degradable layer 211A, and a second supporting filament 211b forms a non-degradable layer 211B. The degradable layer 211A and the non-degradable layer 211B are adjacent to and connected to each other at an adjacent position. In this embodiment, a connection between the absorbable and non-absorbable units is shown in the figure. A tail end of the absorbable filament is provided with an arcuate tube, which is configured to movably connect the non-degradable filament, thereby allowing the arcuate tube and the non-degradable filament to move relative to each other within a certain range, and increasing mesh space. In this way, selection using an internal branch guidewire becomes convenient and compression on a branch stent after implantation is reduced. A proportion of absorbable units is increased.

### Embodiment 9

Referring to FIG. 15(a) to 15(c), in this embodiment adopts a double-layer structure is adopted, that is, a local supporting layer 22 is added. A supporting structure 20 includes a main supporting layer 21 and a local supporting layer 22. The local supporting layer 22 and the main supporting layer 21 are stacked from inside to outside, i.e., the main supporting layer 21 is located outside, the local supporting layer 22 is located inside, and the main supporting layer 21 is located above the local supporting layer 22. A tail end of a non-absorbable filament supporting corrugated ring is connected to a half-corrugated ring to form an integrated structure, thereby enhancing the supporting performance of an end filament and the structural stability of a non-degradable portion. This optimizes a tip structure in Embodiment 3.

According to the vascular stent of the present invention, at least part of the supporting structure at the top of the vascular stent is configured to be degradable. In this way, the initial mechanical supporting effect of a recess section is guaranteed while retaining the overall structure. In a long term, the material of this part can be automatically absorbed after a certain time, thereby reducing the restraint and compression on a branch stent, reducing the occupation of the main lumen space, and reducing irritation to vascular wall.

Each technical feature of the above embodiments may be freely combined. For brief description, not all possible combinations of each technical feature in the abovementioned embodiments are described, but all the combinations of these technical features shall fall within the scope recorded in the present invention without conflicts.

The abovementioned embodiments only express some implementations of the present invention and are specifically described in detail and not thus understood as limits to the patent scope of the present invention. It is to be pointed out that those of ordinary skill in the art may further make a plurality of transformations and improvements without departing from the concept of the present invention and all of these fall within the scope of protection of the present invention. Therefore, the scope of patent protection of the present invention should be subject to the appended claims.

## Claims

1. A vascular stent, comprising a main stent provided with a main lumen, wherein the main stent comprises a proximal section, a distal section, and a middle section located between the proximal section and the distal section and extending axially; the middle section is provided with a recessed portion, above which a supporting structure is arranged; and at least part of the supporting structure is degradable.

2. The vascular stent according to claim 1, wherein the supporting structure is provided with a branch passage that radially penetrates through the supporting structure; the branch passage allows a branch stent to pass through; at least part of an edge of the branch passage is degradable, enabling the branch passage to be expanded upon at least partial degradation; and the expanded branch passage exerts reduced compression on the branch stent passing therethrough.

3. The vascular stent according to claim 2, wherein the branch passage comprises at least a right branch passage near a distal side of the supporting structure; and/or a left branch passage near a proximal side of the supporting structure.

4. The vascular stent according to claim 2, wherein the supporting structure comprises a mesh structure having a plurality of grids, and one or more of the grids form the branch passage.

5. The vascular stent according to claim 1, wherein the supporting structure comprises a main supporting layer that is at least partially degradable; the main supporting layer is formed by supporting filaments; and the supporting filaments comprise at least a degradable first supporting filament.

6. The vascular stent according to claim 5, wherein the degradable first supporting filament forms the main supporting layer.

7. The vascular stent according to claim 5, wherein the supporting filaments further comprise a non-degradable second supporting filament, and the degradable first supporting filament and the non-degradable second supporting filament jointly form the main supporting layer.

8. The vascular stent according to claim 7, wherein
the first supporting filament forms a degradable layer, while the second supporting filament forms a non-degradable layer, and the degradable layer and the non-degradable layer are arranged opposite to each other within a same layer; or
the first supporting filament forms a degradable layer, while the second supporting filament forms a non-degradable layer, and the degradable layer and the non-degradable layer are adjacent to and connected to each other at an adjacent position; or
the first supporting filament and the second supporting filament are arranged in an interlaced manner within a same layer, such that the first supporting filament forms a plurality of degradable layers, while the second supporting filament forms a non-degradable layer, and the plurality of degradable layers are distributed in the non-degradable layer within the same layer.

9. The vascular stent according to any one of claims 6 to 8, wherein during the formation of the main supporting layer, the plurality of supporting filaments are intersected and form intersection points; and at the intersection points, the plurality of supporting filaments are interconnected, and/or overlapped, and/or hooked.

10. The vascular stent according to claim 9, wherein
when the plurality of supporting filaments are overlapped at the intersection points, the plurality of supporting filaments at the intersection points may be connected via a fastener and the fastener is configured to radially restrain the plurality of supporting filaments to avoid radial separation from one another; and/or
when the plurality of supporting filaments comprise the first supporting filament and the second supporting filament, and the first supporting filament and the second supporting filament are overlapped at the intersection points, the first supporting filament at the intersection points is located above the second supporting filament; and/or
when the plurality of supporting filaments comprise the first supporting filament and the second supporting filament, and the first supporting filament and the second supporting filament are overlapped at the intersection point, a radial thickness of the first supporting filament at least at the intersection point is greater than a radial thickness of the second supporting filament, and a cross passage through which the second supporting filament passes is formed at the intersection point of the first supporting filament.

11. The vascular stent according to claim 10, wherein
when the supporting filaments at the intersection points are connected with each other via the fastener, a first sliding passage extending in a first predetermined direction and a second sliding passage extending in a second predetermined direction are formed on the fastener; one of the supporting filaments slides relative to the fastener along the first predetermined direction, while the other supporting filament slides relative to the fastener along the second predetermined direction, and the first predetermined direction and the second predetermined direction are predetermined movement directions of the supporting filaments when the vascular stent is compressed or self-expands; and/or
when the cross passage through which the second supporting filament passes is formed on the first supporting filament, the cross passage has a predetermined length along an extension direction of the first supporting filament.

12. The vascular stent according to claim 9, wherein when the plurality of supporting filaments are hooked, an elastic connecting member which extends axially is arranged between the supporting filaments at a hooking position; end portions of the elastic connecting member are respectively connected to hooked portions of the plurality of supporting filaments; and the elastic connecting member is configured to elastically pull the supporting filaments toward each other.

13. The vascular stent according to claim 6 or 7, wherein the supporting structure further comprises a non-degradable local supporting layer; the local supporting layer and the main supporting layer are stacked from inside to outside; and the local supporting layer is opposite to a degradable layer that is formed by the first supporting filament and that is located in the main supporting layer.

14. The vascular stent according to claim 5, wherein the main supporting layer comprises a mesh structure; in a naturally expanded state, the main supporting layer comprises, in a circumferential direction, a first mesh region and a second mesh region connected to the first mesh region; the first mesh region comprises a plurality of rows of intersection units which are formed by mutually overlapping a plurality of first-direction supporting filaments arranged at intervals and a plurality of second-direction supporting filaments arranged at intervals; the second mesh region comprises at least one row of hooking units, each row of hooking units comprises one or more axially arranged hooking units, and each hooking unit comprises a first hooking member and a second hooking member; and in at least part of the hooking units, the first hooking member and the second hooking member are substantially axially hooked, alternatively, in at least part of the hooking units, the first hooking member and the second hooking member are substantially axially separated to form a gap, alternatively, in at least part of the hooking units, the first hooking member and the second hooking member are abutted against each other in a non-hooking manner.

15. The vascular stent according to claim 5, wherein the degradable first supporting filament is either entirely degradable or partially degradable; and when the first supporting filament is partially degradable, the first supporting filament has a multi-layer structure arranged from inside to outside, and comprises a non-degradable inner core located at an innermost layer and a degradable covering layer located at an outermost layer.
